# EUROPEAN PATENT APPLICATION

(11) **EP 2 093 011 A1**
(43) Date of publication of application: **26.08.2009**
(21) Application number: 08151847.4
(22) Date of filing: 22.02.2008
(51) Int. Cl.: B23K 26/03, B23K 26/38, A61M 25/09, A61F 2/90

(54) **A method and apparatus for detecting hole breakthrough in a laser drilling process, using an optical fibre**

(71) Applicant: National University of Ireland Galway, Galway (IE)
(72) Inventor: Flaherty, Tony, Galway (IE); Bernard, Fabien, Galway (IE)
(74) Representative: Lane, Cathal Michael

(57) **Abstract**

The present invention provides a system for monitoring breakthrough of a drilling process of a drilling laser through a wall of a target sample (3). The system comprises an optical fiber (1) locatable perpendicular to the optical path of the drilling laser and adjacent the sample (3) so that the wall (13) to be drilled is positioned between the laser and the fiber (1) and a detector (16) arranged to detect when light which has been emitted radially by the fiber (1) passes through the wall (13) of the sample (3) during the drilling process.

## Description

### Field of the Invention

The present invention relates to a method and apparatus for detecting hole breakthrough in a laser drilling process. More particularly, the invention relates to the detection of hole breakthrough by the use of an optical detection system.

### Background to the Invention

The drilling of holes through tubes of narrow bore is common practice throughout a number of industrial fields. One industry sector which makes use of narrow tubes with drilled holes is the medical device field. Some medical devices require a large number of holes to be drilled in the walls of a catheter or hypotube. Often these holes are created using a laser.

Catheter tubes are usually long and are of narrow-bore (in the millimeter range), with the holes being small, typically within the range 1-100 microns. This particular hole geometry makes it difficult to inspect holes, and therefore makes confirmation of breakthrough extremely problematic i.e. the instant in which a drilled hole is first opened.

Failing to detect breakthrough can lead to reductions in process yield and efficiency. Furthermore, in the case of a small-bore tube sample, after breakthrough has occurred, the backwall opposite to the drilled hole can become damaged, as the laser penetrates through the open hole and impacts the backwall. It will therefore be appreciated that it is important to detect breakthrough as quickly as possible.

One existing method of breakthrough detection uses a pin gauge. However, the pin gauge's ability to detect breakthrough is limited, as they are only available in sizes down to approximately 70 microns , and this is insufficient precision for a wide range of applications. Furthermore, many of the integral features of existing pin gauges tend to be delicate and can be easily damaged. Delicate pin gauges frequently need to be repaired and replaced, which can be expensive. In addition, the method of detecting hole breakthrough using pin gauges can be very time consuming and labour intensive, especially for an array of holes. The use of a pin gauge generally requires each hole to be individually located, the pin pushed into the hole and then mechanically, or manually moved to the next location. Furthermore, the use of a pin-gauge is incompatible with real-time breakthrough sensing.

Another method of detecting hole breakthrough involves the use of airflow measurements. However the results in certain cases may not be reliable. For example, in products where a large number of holes have been drilled, the results from this technique can be ambiguous, as the airflow measurement allows only the determination of the total area drilled, and not the sizes of individual machined features. Therefore, a small increase in the drilled diameter of some holes can sometimes mask the presence of blocked or incomplete holes.

Existing methods can also have difficulty in detecting breakthrough in holes positioned at awkward positions. Long narrow holes positioned in 'difficult to reach' locations may not be analysed using pin gauges, and the ambiguous airflow methods do not provide an adequate solution.

In light of the above problems, there is a need in the art for a quick, reliable breakthrough detection technique that is capable of testing breakthrough in any number of holes, including those positioned in less than ideal positions and orientations, and in long tubes of narrow bore.

There is also a need for a breakthrough detection technique which does not physically stress the device through either contact (for example mechanical gauge pins) or pressure (airflow measurement systems).

There is also a need to provide an offline detection tool that can be used to determine whether the required number of holes have been successfully drilled by a drilling laser.

### Summary of the Invention

The present invention provides a system for monitoring breakthrough of a drilling process of a drilling laser through a wall of a target sample. The system comprises:
An optical fiber locatable perpendicular to the optical path of the drilling laser and adjacent the sample so that the wall to be drilled is positioned between the laser and the fiber, and a detector arranged to detect when light which has been emitted radially by the fiber passes through the wall of the sample during the drilling process.

The location of an optical fiber in this manner, and the provision of a detector which can detect when light which has been emitted radially by the fiber passes through the wall of the sample, provides an efficient and accurate means of detecting hole breakthrough for samples of all sizes.

The optical fiber may be a side-emitting optical fiber.

The detector may be located adjacent the end facet of the fiber so as to detect light from the drilling laser which penetrates the wall of the sample upon breakthrough and is absorbed by the fiber and transmitted to its end facet.

The system may further comprise an illumination source arranged so as to direct light into the fiber via its end facet, and wherein the detector is arranged to detect when the light from the illumination source is radially emitted by the fiber and passes through the wall of the sample upon breakthrough.

The system may further comprise a directing means located in the optical path of the drilling laser, wherein the directing means directs the radially emitted light passing through the wall of the sample to the detector.

The directing means enables only light which has been radially emitted from the fiber through the wall of the sample into the light detection system to be reflected, while allowing the light from the laser to pass through unaffected.

The detector may generate an indicator when light passing through the wall of the sample is detected.

The indicator may be a control signal to the drilling laser to stop the laser drilling.

Alternatively, the indicator may be a visual or an aural indicator that breakthrough has been detected.

The target sample may be a narrow tube.

Preferably, the fiber may be located within the channel of the tube.

The location of the fiber within the channel of the tube minimizes the damage to the backwall of the tube which can occur after breakthrough has occurred.

The detector may be one of: a CCD camera, a CMOS camera or a photodiode.

The system may further comprise an imaging system located in front of the detector.

The imaging system may comprise an optical microscope.

The directing means may be one of: a beam splitter, a dichroic mirror or a spatial filter.

The detector may further comprise image processing software, and wherein the detector creates an image of the light it captures, which may then be analysed by the image-processing software to determine whether light which has been emitted radially by the fiber has passed through the wall of the sample during the drilling process.

### The system may further comprise a drilling laser.

The present invention also provides a method of detecting breakthrough through a wall in a target sample to be drilled by a drilling laser comprising the steps of:
locating an optical fiber perpendicular to the optical path of the laser and adjacent the sample, so that the wall to be drilled is positioned between the laser and the fiber, drilling the wall of the target sample; and
detecting when light which has been emitted radially by the fiber passes through the wall of the sample during the drilling process.

A side emitting fiber may be located perpendicular to the optical path of the laser.

The detection may be performed adjacent the end facet of the fiber so as to detect light from the drilling laser which penetrates the wall of the sample upon breakthrough and is absorbed by the fiber and transmitted to its end facet.

The method may further comprise the step of directing light into the fiber via its end facet prior to drilling the wall of the target sample, and wherein that directed light which is radially emitted by the fiber and passed through the wall of the sample upon breakthrough is detected.

The method may further comprise generating an indicator when light passing through the wall of the sample is detected.

The step of detecting may comprise the steps of: creating an image of the light and analysing the image using image-processing software to determine whether light which has been emitted radially by the fiber has passed through the wall of the sample during the drilling process.

The method may further comprise the step of magnifying the light prior to creating an image of the light.

The present invention also provides a system for determining whether a drilling laser has completed a hole through the wall of a target sample, the system comprising:
an optical fiber locatable adjacent the intended location of the hole in the wall of the target sample and perpendicular to the optical path taken by the drilling laser when the hole was to be drilled;
an optical source located so as to direct light into the end facet of the fiber; and
a detector for detecting light from the optical source radially emitted by the fiber passing through the intended location of the hole.

The optical fiber may be a side emitting fiber.

### Brief Description of the Drawings

The invention will be more clearly understood from the following description of embodiments thereof, given by way of example only, with reference to the accompanying drawings, in which:
Figure 1 shows a first embodiment of the method and apparatus of the present invention; and
Figure 2 shows an alternative embodiment of the method and apparatus of the invention.

### Detailed Description of the Drawings

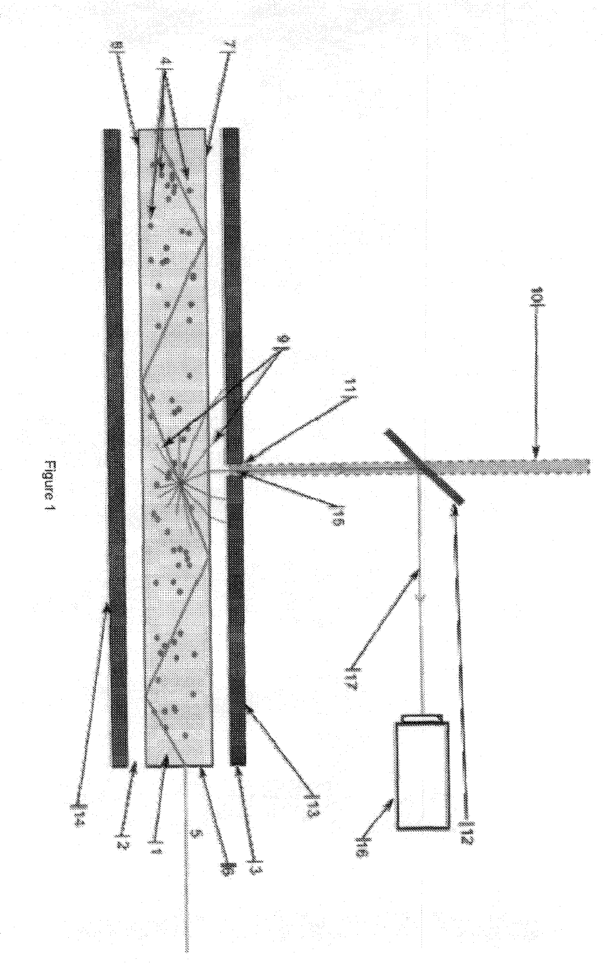

Figure 1 shows a first embodiment of the apparatus of the present invention used to detect breakthrough in a laser drilling process. A target sample to be drilled is in the form of a catheter 3 having a hollow channel, or lumen 2. This channel 2 may be of narrow bore. An optical source 10 in the form of a drilling laser is provided for the drilling of holes in the wall 13 of the catheter 3. An optical fiber in the form of a side emitting fiber 1 is positioned inside the hollow channel 2, so that it is located perpendicular to the optical path of the drilling laser 10 and adjacent the catheter 3 so that the wall 13 to be drilled is positioned between the laser 10 and the side emitting fiber 1.

An illumination light source 5 is positioned adjacent an end facet of the fiber 1 and adapted to direct light into the fiber 1. In the described embodiment, the light source 5 takes the form of a laser or a LED.

A light detection system 16 is adapted to detect light emitted from the fiber 1. The detection system may be any suitable means for detection of optical rays, such as a CCD, a CMOS camera or a photodiode and image processing software. The detection system is positioned so as to capture light beams 17 which have been emitted radially by the fiber 1 and passed through the wall 13 of the sample 3. In the described embodiment, a directing means 12 is also provided, and positioned in the optical path of the drilling laser 10, in order to direct light into the light detection system 16. The purpose of the directing means 12 is to reflect only light radially emitted from the fiber 1 through the wall 13 of the sample 3 into the light detection system 16, while allowing the light from the laser 10 to pass through unaffected. This may be achieved for example using a reflector whose performance varies with wavelength, known as a dichroic mirror, or using a mirror with a small hole in its centre, known as a spatial filter. In one embodiment of the invention, an imaging system in the form of an optical microscope is located in front of the camera, in order to magnify the light prior to detection by the detection system 16.

The side-emitting fiber 1 differs from a transmission fiber optic by the light leakage through its walls. Instead of guiding most of the incoming light from one end of the fiber to the other, as is the case in traditional optical fibers, a side-emitting fiber 1 allows the light to escape radially over a certain length.

A side emitting fiber can be constructed in a variety of ways. One method involves designing the fiber to allow leakage of light at the interface between the fiber core and its outer core. Another method involves providing the fiber with a plurality of scattering centres 4 distributed throughout the length of the fiber 1, so that light will be scattered when it impacts the scattering centres. The scattering centres can be created in the optical fiber 1 by the appropriate selection of the refractive index of the fiber cladding relative to that of the core, causing the light to leak through the cladding. Another technique of achieving scattering centres is by providing the core of the fiber with microbends, so that the transmitted optical beam will meet areas locally not meeting the condition for total internal reflection, in order that scattering centres will occur at each microbend of the core. In a third technique, fluorescent or scattering particles can be added to either the core or the cladding of the fiber during its extrusion.

In order to detect breakthrough in the laser drilling process on the catheter, light from the illumination light source 5 should first be directed into the side-emitting fiber 1. As the light 5 passes through the optical fiber 1, it will travel along the length of the fiber, reflecting between the fiber sidewalls 7 and 8, and emitting radially from the fiber as it interacts with the scattering centres 4.

Once the light from the light source 5 has been directed into the side emitting fiber 1, the drilling process can begin, by focussing the drilling laser 10 at the desired drilling point 11 of a hole in the wall 13 of the target sample (in this case the catheter tube 3), and powering it on, so that it emits a laser beam directed on the drilling point 11.

When breakthrough occurs, light from the light source 5 which is being radially emitted by the fiber 1 exits the catheter 3 through the drilled hole 15 in the wall 13 of the target sample 3. This light exiting the catheter 3 originating from the illumination light source 5 is detected by the light detection system 16 either directly, or via the directing means 12. This light may optionally have been magnified first by an optical microscope located in front of the detection system 16. Once the light radially emitted from the fiber 1 is detected by the detection system 16, it signifies that breakthrough has occurred, and accordingly that the drilling should be stopped The detection system 16 can signal that the drilling should be stopped by the use of an indicator, such as a visual or an aural indicator. Alternatively, a control "off" signal from a control processor, which takes a signal from the detector 16, may be automatically sent to the drilling laser 10, or to an external device, to stop the drilling. It will be appreciated that the processing of light captured by the detector can be performed in a number of different ways. The detector may for example create an image of the light it captures, which is then analysed by the image-processing software to determine whether breakthrough has in fact occurred.

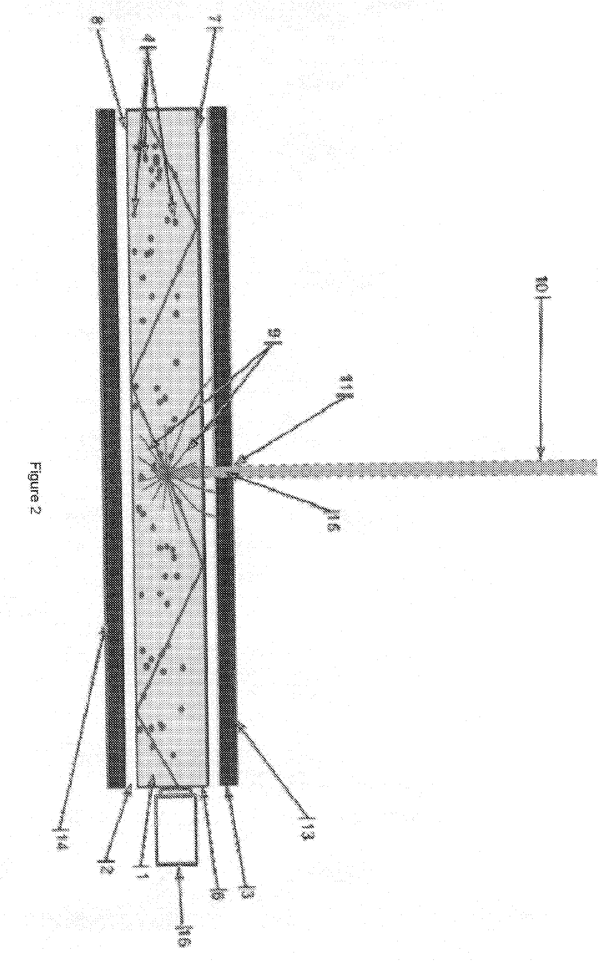

Figure 2 details an alternative embodiment of the invention. The same reference numerals are used in respect of the same components as that of the first embodiment. In contrast to the first embodiment, the detecting means 16 is now placed at the end facet 6 of the side emitting optical fiber 1. In this second embodiment, upon breakthrough, the light from the drilling laser 10 penetrates the wall 13 of the catheter 3 and is absorbed by the fiber 1 and transmitted along its length to its end facet. The light transmitted along the fiber is then detected by the light detection system 16. This light may again optionally have been magnified first by an optical microscope located in front of the detection system 16. It will be noted that in this embodiment no separate illumination light source is necessary, as breakthrough is detected when absorbed light from the drilling laser 10 is detected by the detecting means 16 at the end facet 6 of the fiber 1.

It will be appreciated that after breakthrough has occurred, and in the absence of a 'laser off' signal, the drilling laser 10 will continue to machine the wall 13 of the catheter 3. On penetration of the wall 13, the drilling laser 10 will enter the channel or lumen 2 of the catheter and will impact the side emitting fiber 1. If the fiber were not present, the drilling laser would impact the back wall 14 of the catheter 3, causing undesirable damage to the back wall 14. However, in the arrangements described above, the wall 14 is protected against the drilling laser 10 impact by the side emitting fiber 1. On impacting the side emitting fiber 1 inside the catheter channel 2, the laser beam will be scattered, causing the laser beam's energy to dissipate. The fiber will also absorb some of the energy of the drilling laser beam. The degree of scattering and absorption will be such that the remaining energy in the dispersed laser beams will be below the threshold value required to cause damage to the catheter wall. Therefore, placing the side emitting fiber 1 within the tube 3 has the added advantage of protecting the back wall from drilling damage, by scattering and absorbing the drilling laser beam once breakthrough on the front wall 13 has occurred, and before the detection system 16 has detected that breakthrough has occurred and any indicator or laser off signal is generated.

In addition to using the system as a breakthrough sensor, the side emitting fiber system may also be used in process control, as an offline measurement tool. This is achieved by placing a side emitting optical fiber 1 adjacent the tube 3 at those locations where holes were intended to be present after the drilling process, and perpendicular to the optical path which would have been taken by the drilling laser when the holes were being drilled. A detector 16 and an illumination source 5 should also be located in the same positions as those shown in Figure 1. When the optical fiber 1 is illuminated by the illumination source 5, the radially emitted light will pass through any completed holes in the tube 3. The detector 16 can then capture the image, while the image-processing software will automatically detect and count the number of completed holes. This light may optionally have been magnified first by an optical microscope located in front of the detector 16. It will be appreciated that the hole shape and size, and the location of the holes, can also be analysed using pattern-recognition algorithms within the image processing software.

The present invention provides numerous advantages over the prior art techniques of detecting hole breakthrough. It provides an efficient and accurate means of detecting hole breakthrough. In contrast to prior art techniques, the apparatus of the present invention can also be used to detect holes of diameters within the range 1-10000 microns. Furthermore, it can be used to detect large numbers of bored holes. It can also detect holes that are to be machined in difficult to access locations on a sample. Furthermore, it minimizes the damage to the backwall of a catheter which can occur after breakthrough has occurred.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A system for monitoring breakthrough of a drilling process of a drilling laser through a wall of a target sample comprising:
an optical fiber locatable perpendicular to the optical path of the drilling laser and adjacent the sample so that the wall to be drilled is positioned between the laser and the fiber, and a detector arranged to detect when light which has been emitted radially by the fiber passes through the wall of the sample during the drilling process.

2. The system of Claim 1 wherein the optical fiber is a side-emitting fiber.

3. The system of Claim 1 or Claim 2 wherein the detector is located adjacent the end facet of the fiber so as to detect light from the drilling laser which penetrates the wall of the sample upon breakthrough and is absorbed by the fiber and transmitted to its end facet.

4. The system of Claim 1 or Claim 2, further comprising an illumination source arranged so as to direct light into the fiber via its end facet, and wherein the detector is arranged to detect when the light from the illumination source is radially emitted by the fiber and passes through the wall of the sample upon breakthrough.

5. The system as claimed in Claim 4, further comprising a directing means located in the optical path of the drilling laser, wherein the directing means directs the radially emitted light passing through the wall of the sample to the detector.

6. The system as claimed in any of the preceding claims, wherein the detector generates an indicator when light passing through the wall of the sample is detected.

7. The system of Claim 6, wherein the indicator is a control signal to the drilling laser to stop the laser drilling.

8. The system of Claim 6, wherein the indicator is a visual or an aural indicator that breakthrough has been detected.

9. The system as claimed in any of the preceding claims, wherein the target sample is a narrow tube.

10. The system of claim 9 wherein the fiber is located within the channel of the tube.

11. The system as claimed in any of the preceding claims wherein the detector is one of: a CCD camera, a CMOS camera or a photodiode.

12. The system as claimed in claim 11, further comprising an imaging system located in front of the detector.

13. The system as claimed in claim 12, wherein the imaging system comprises an optical microscope.

14. The system as claimed in any of Claims 5 to 13, wherein the directing means is one of: a beam splitter, a dichroic mirror or a spatial filter.

15. The system as claimed in Claim 11, wherein the detector further comprises image processing software, and wherein the detector creates an image of the light it captures, which is then analysed by the image-processing software to determine whether light which has been emitted radially by the fiber has passed through the wall of the sample during the drilling process.

16. The system as claimed in any of the preceding claims further comprising a drilling laser.

17. A method of detecting breakthrough through a wall in a target sample to be drilled by a drilling laser comprising the steps of:
locating an optical fiber perpendicular to the optical path of the laser and adjacent the sample, so that the wall to be drilled is positioned between the laser and the fiber, drilling the wall of the target sample; and
detecting when light which has been emitted radially by the fiber passes through the wall of the sample during the drilling process.

18. The method as claimed in Claim 17, wherein a side emitting fiber is located perpendicular to the optical path of the laser.

19. The method of Claim 17 or Claim 18, wherein the detection is performed adjacent the end facet of the fiber so as to detect light from the drilling laser which penetrates the wall of the sample upon breakthrough and is absorbed by the fiber and transmitted to its end facet.

20. The method of any of claims 17 to 19, further comprising the step of directing light into the fiber via its end facet prior to drilling the wall of the target sample, and wherein that directed light which is radially emitted by the fiber and passed through the wall of the sample upon breakthrough is detected.

21. The method as claimed in any of Claims 17 to 20, further comprising generating an indicator when light passing through the wall of the sample is detected.

22. The method as claimed in any of claims 17 to 21, wherein the step of detecting comprises the steps of: creating an image of the light and analysing the image using image-processing software to determine whether light which has been emitted radially by the fiber has passed through the wall of the sample during the drilling process.

23. The method as claimed in claim 22, further comprising the step of magnifying the light prior to creating an image of the light.

24. A system for determining whether a drilling laser has completed a hole through the wall of a target sample, the system comprising:
an optical fiber locatable adjacent the intended location of the hole in the wall of the target sample and perpendicular to the optical path taken by the drilling laser when the hole was to be drilled;
an optical source located so as to direct light into the end facet fiber; and
a detector for detecting light from the optical source radially emitted by the fiber passing through the intended location of the hole.

25. The system of Claim 24, wherein the optical fiber is a side emitted fiber.
